# EUROPEAN PATENT APPLICATION

(11) **EP 1 972 640 A1**
(43) Date of publication of application: **24.09.2008**
(21) Application number: 07450062.0
(22) Date of filing: 23.03.2007
(51) Int. Cl.: C07K 16/42, C12N 5/20, C12N 15/13

(54) **Apoptosis inducing antibodies**

(71) Applicant: BIOMAY AG, 1090 Wien (AT)
(72) Inventor: The designation of the inventor has not yet been filed
(74) Representative: Sonn & Partner Patentanwälte

(57) **Abstract**

The present invention describes an anti-mIg-antibody which binds specifically to the membrane-vicinal region (MVR) of the extracellular membrane-proximal domain (EMPD) of membrane bound human immunoglobulins (mIgs).

## Description

The present invention relates to anti-mIg-antibody, their pharmaceutical use and methods of producing such antibodies.

Immunoglobulins (Igs) can be found in two forms. In the secreted form (sIg), they represent the effector arm of the humoral system. They can also be expressed on the surface of a B cell in a membrane-bound form (mIg), and, in this physical state, they can convey signals to steer the B cell along its differentiation pathway. The sIgs differ from the mIgs by lacking the membrane anchoring polypeptide comprising an extracellular membrane-proximal domain (EMPD), a transmembrane segment which is about 25 amino acid in length and has the potential to interact with other polypeptides (it behaves like a classical antigen receptor on B lymphocytes) and a cytoplasmic domain differing in size and function in different mIg subclasses. For example, in mIgM and mIgD, the cytoplasmic domain is only three amino acids long (KVK) whereas in other mIg classes there are usually 14 to 28 amino acid long cytoplasmic domains. The EMPD differs significantly in length and sequence between the different Ig isotypes and different species. For example, human mIgE has i.a. two isoforms of this EMPD, one being 15 amino acids in length, the other having an additional 52 amino acid extra domain (Inführ et al., Allergy 60 (2005), 977-985; Geisberger et al., Immunology 118 (2006), 429-437; Chen et al., Int. Arch. Allergy Immunol. 128 (2002), 315-324).

Allergic diseases and conditions, such as asthma, allergic rhinitis, atopic dermatitis, and food allergy, have become increasingly prevalent over the past few decades and now affect 10-40% of the population in industrialized countries. Although most cases of allergic disease are not life-threatening, some allergic conditions can result in serious acute conditions or even death. However, quality of life is usually profoundly affected. Allergies are also the largest cause of time lost from work and school, and their impact on personal lives as well as their direct and indirect costs to the medical systems and economy are enormous.

Most allergic diseases are caused by IgE-mediated hypersensitivity reactions. IgE, a class of antibody normally present in the serum at minute concentrations, is produced by IgE-secreting plasma cells, which express the antibody on their surface at a certain stage of their maturation. For reasons still not well understood, allergic patients produce increased amounts of IgE with binding specificity for ordinarily innocuous antigens to which they are sensitive. These IgE molecules circulate in the blood and bind to IgE-specific receptors on the surface of basophils in the circulation and mast cells along mucosal linings and underneath the skin. In an allergic reaction, the inhaled or ingested allergens bind to IgE on mast cells or basophils, cross link the IgE molecules, and aggregate the underlying receptors, thus triggering the cells to release histamine and the other pharmacological mediators of the symptomatic allergic response.

Current drugs for allergic diseases, such as antihistamines, corticosteroids, and bronchodilators (J3-adrenergic receptor agonists), treat allergic symptoms and concomitant inflammatory reactions. Desensitization immunization with antigens (allergens), which is used mainly in the United States for allergic rhinitis, is not effective for about half of the treated patients. Therefore, a treatment that targets the allergic process, prevents it from occurring, and has fewer side effects than current drugs is desirable. Because IgE is the central macromolecular mediator responsible for the progression of allergic reactions, neutralizing it and inhibiting its synthesis would appear to be a rational approach for the treatment of various allergic diseases (Chang et al., Nat. Biotech. 18 (2000), 157-162). This promising concept for the treatment of allergy involved the application of monoclonal antibodies, which are IgE isotype-specific and are thus capable of binding IgE. This approach was based on the inhibition of allergic reactions by down regulating the IgE immune response, which is the earliest event in the induction of allergy and provides for the maintenance of the allergic state. As the response of other antibody classes is not affected, both an immediate and a long lasting effect on allergic symptoms should be achieved.

This therapeutic approach, based on humanised anti-IgE monoclonal antibodies of a particular and unique specificity (abbreviated here as anti-IgEs), for treating IgE-mediated diseases has already been commercially developed. In one program, CG-P5I9OI, a chimeric IgGl antibody, went through phase and phase I clinical trials for allergic rhinitis and allergic asthma. A "switchover" phase I trial was performed using CGP56901, a humanised antibody derived from the same mouse antibody. In a separate program, rhuMAb-E25, a humanised antibody derived from a similar mouse antibody, went through several phase I and phase II trials on allergic rhinitis and asthma. After the two programs were combined in 1996, rhuMAb-E25 was chosen for further clinical development. One pivotal phase IIb and one phase III trial on allergic rhinitis, and phase III trials on adult and paediatric asthma have been completed, all with positive results. Recently, a phase II trial on peanut-induced hypersensitivity was initiated with anti-IgE to evaluate its efficacy in preventing food-induced allergy and anaphylactic reactions (Chang et al. (2000)).

However, this treatment concept is connected to major problems and risks: First, there is the risk of long-term depletion of IgE, which can compromise defence against certain infections, such as parasites, or postulated roles in immune surveillance for malignant cells. Second, even if IgE is eliminated almost entirely (i.e. to an extent of 99%), the therapy could still not work as intended, because only a very few IgE molecules on mast cells or basophils are sufficient to sensitize the cell. For this approach, specifically down-regulating IgE production by B cells would be desirable compared to inhibiting or neutralising free IgE. Third, immune complexes, such as the ones formed by IgE and the anti-IgE antibody are generally considered harmful.

On the other hand, it has also been postulated that anti-IgE binds to mIgE on IgE-expressing B cells, and as mIgE is a part of the B-cell receptor, anti-IgE may inhibit the B cells or even cause their lysis, like anti-IgM or anti-IgG (Warner et al., J. Immunol. 146 (1991), 2185-2191). However, the antibodies with the specificity against the EMPD according to Chen et al. (2002) have been found to be unable to cause apoptosis in IgE expressing B cells (Poggianella et al., J. Immunol. 177 (2006), 3597-3605). It was therefore not possible to provide antibodies which induce apoptosis; there was no suitable prediction method available in the art for such functional antibodies.

There is, however, the need for a specific immunotherapy targeting the Ig producing B cells, without affecting free Igs in order to specifically target the source of the Ig producing B cell, especially in an isotope specific manner. Such a method could be used in many medical fields, including immunosensibilisation, treatment of autoimmune diseases and treatment of cancer, especially leukaemias.

Accordingly, it is an object of the present invention to provide such a specific immunotherapy targeting method.

Therefore, the present invention provides an anti-mIg-antibody which binds specifically to the membrane-vicinal region (MVR) of the extracellular membrane-proximal domain (EMPD) of membrane bound human immunoglobulins (mIgs). The antibodies according to the present invention are specific for binding of mIgs, they do not bind to the soluble forms thereof and have no binding capacity to Fc_{ε}RI or Fc_{ε}RII. In contrast to other anti-Ig antibodies, these antibodies according to the present invention surprisingly have the ability to produce apoptosis of specific B cells, i.e. of those cells which produce the membrane bound antibodies which specifically bind to the antibodies according to the present invention. The membrane-vicinal region (MVR) to which the antibodies according to the present invention bind is defined in general as the region extending up to a maximum of 25 amino acid residues. Preferred antibodies bind in the region extending up to 20 amino acid residues, especially up to 16 amino acids, into the extracellular space from the transmembrane sequence of mIgs. Binding of the antibodies to this region has been identified as an essential prerequisite for the apoptosis inducing ability of such antibodies. In fact, the antibodies according to the present invention have an apoptose inducing capacity on their own without being dependent on complement mediation; the antibodies according to the present invention can therefore also be defined as being able to elicit a "non-complement-mediated" apoptosis.

Surprisingly, the antibodies according to the present invention are superior compared to antibodies binding to extracellular regions being more distant from the transmembrane domain. For example, antibodies binding to the long form of mIgE-EMPD are not able to induce apoptosis by their own, only "complement-mediated" apoptosis, which is, of course not suitable for an effective treatment. Such antibodies therefore have not the capability of the antibodies according to the present invention.

The antibodies according to the present invention are preferably provided as humanised antibodies. Antibodies which are originally provided not of human origin are less suitable for application to humans due to their immunogenicity as foreign proteins. This immunogenicity can be reduced by transforming, e.g., a rodent anti-IgE monoclonal antibody into a chimeric antibody which combines the variable domains of the rodent antibody with human antibody constant domains. This approach conserves the antigen-binding site of the rodent parent anti-IgE antibody, while conferring the human isotype and effector functions. The immunogenicity of a chimeric antibody can be further reduced by grafting rodent hypervariable regions, also termed complementarity determining regions (CDRs), into the frameworks of human light and heavy chain variable region domains resulting in reshaped human antibodies. The technique involves the substitution or recombinant grafting of antigen-specific rodent CDR sequences for those existent within "generic" human heavy and light chain variable domains (e.g. US 6,180,370).

According to the present invention the antibodies can be monoclonal antibodies (including full length monoclonal antibodies), polyclonal antibodies, and multispecific antibodies (e.g., bis-pecific antibodies). The antibodies according to the present invention also include antibody fragments, such as Fab, Fab', F(ab')₂, single-chain Fv (sFv) and Fv fragments as long as they have the qualitatively the same binding specificity as the full antibody (i.e. specifically binding the mIg and producing apoptosis in B cells). Additional chemical couplings of antibody fragments are known to those of ordinary skill in the art.

The preferred humanised forms of non-human (e.g. murine) antibodies are chimeric immunoglobulins, immunoglobulin chains or fragments thereof (such as Fv, Fab, Fab', F(ab')₂ or other target-binding sub-sequences of antibodies) which contain minimal sequence derived from non-human immunoglobulin. In general, the humanised antibody comprises substantially all of at least one, and typically two, variable domains, in which all or substantially all of the CDR regions correspond to those of a non-human immunoglobulin and all or substantially all of the FR regions are those of a human immunoglobulin consensus sequence. The humanised antibody may also comprise at least a portion of an immunoglobulin constant region (Fc), typically that of a human immunoglobulin template chosen.

The techniques for providing antibodies in polyclonal or monoclonal form, especially in humanised form, are well available to the man skilled in the art and e.g. also described in WO 2005/075504 A1).

Currently there is no isotype specific memory cell- antibody available. The antibodies according to the present invention allow such a memory cell-specific detection. Therefore, the present invention provides isospecific murine anti-mIgE antibodies specifically reactive against the murine EMPD. More specifically, the present invention preferably also relates to the murine antibodies according to the present invention. These antibodies can preferably be used as research tools in mice research or as screening tool for antibody binding compounds.

The antibodies according to the present invention have a specificity to the MVR of the EMPD of migs, especially to the short isoform (Sh) of mIgEs. Preferred antibodies according to the present invention have an affinity to the mIg of at least 10⁻⁸ M, especially of at least 10⁻⁹ M. These high-affinity antibodies according to this preferred embodiment have a high therapeutic potential due to their binding capacities, especially with respect to their specific B cell apoptotic characteristics.

In principle, the present invention is applicable to all isoforms of migs. Therefore, the present invention preferably relates to anti-mIg-antibodies which specifically bind to mIgA, mIgG, mIgE or mIgM, especially to mIgE.

The preferred embodiment of the antibody according to the present invention is an anti-human mIgE-antibody as defined herein. This antibody is specifically advantageous due to its isoform-specificity and its apoptosis inducing capability in B cells.

Accordingly, another aspect of the present invention relates to the use of an antibody according to the present invention for the preparation of a medicament causing the apoptosis of B cells.

The present invention is therefore specifically suitable for the preparation of a medicament for a specific immunotherapy, especially for preventing allergic reactions.

The present invention is preferably used in the course of treatment or prevention schemes of IgE-mediated disorders, i.e. conditions or diseases which are characterized by the overproduction and/or hypersensitivity to the immunoglobulin IgE. These disorders include conditions associated with anaphylactic hypersensitivity and atopic allergies, including for example: asthma, allergic rhinitis & conjunctivitis (hay fever), eczema, urticaria, atopic dermatitis, and food allergies. The serious physiological condition of anaphylactic shock caused by, e.g., bee stings, snake bites, food or medication, is also encompassed. The antibodies according to the present invention can preferably used to prevent such conditions or to assist in established treatments (for taking care of the cellular basis of the IgE production in B cells).

IgE raised against normally innocuous environmental allergens is the key effector molecule in allergic diseases. In sensitized atopic individuals, allergen exposure induces cross-linking of high-affinity FcεRI receptor-bound IgE on effector cells and, thus, immediate release of anaphylactogenic mediators. As already mentioned above, like other immunoglobulins, IgE consists of two light chains and two heavy chains (ε-isotype) and can be produced in two forms by alternative splicing: a secreted and a membrane-bound form. Membrane IgE (mIgE) is a transmembrane protein which behaves like a classical antigen receptor on B lymphocytes. Previous reports showed that the expression of a functional membrane form is essential for generating humoral isotype-specific IgE and IgG1 responses in mice, and an IgG2a response in man. According to the present invention it was concluded that the antigen receptor is the only device for an effective antigen presentation and signals that are generated by the receptor are needed not only for the maturation process but also for the expansion of antigen specific cells.

Based on the results provided with the present invention, the present invention provides a suitable approach for interfering with IgE production in vivo. Therefore, the 19 amino acid isotype EMPD and its mammalian, especially murine and human counterparts (its homologues) are provided as the target for the interference with respect to mIgEs and the B cells producing these molecules.

This interference according to the present invention resembles the "first signal" of B cell activation and corresponding B cells rapidly became anergic or apoptotic because the "second signal", the physical linkage to the corresponding T cell, is missing. Thus, the tolerance-inducing mechanism stop B cells in their proliferation and differentiation either through clonal deletion (apoptosis) or through anergy induction. Clonal deletion is the physical elimination (death) of a B cell, while clonal anergy is the silencing of a B cell. Anergic B cells while still alive, do not longer produces antibody, nor can be readily activated. Both mechanisms and/or strategies naturally occur during B cell development and have intensively been studied in immature B cells in the bone marrow or naïve mature B cells.

More specifically, the treatments according to the present invention are highly suitable for the prevention of side effects of treatments which are known to cause a higher sensibilisation. Therefore, the present invention preferably relates to the use of an antibody according to the present invention for the preparation of a medicament for prevention of sensibilisation side effects of a anti-acidic treatment, especially for gastric or duodenal ulcer or reflux.

Alternatively, the antibodies according to the present invention can also be used for the preparation of a medicament for the treatment of autoimmune diseases. In this case, the MVRs of the extramembrane domains of IgM, IgD, IgG1,2,3,4 and IgA are advantageously used. In the treatment of rheumatoid diseases selective immunoblockade is increasingly used. E.g. an anti-TNF-therapy or a treatment with an anti-CD20-antibody can be combined with the administration of an antibody according to the present invention. For example, Rituximab, a monoclonal antibody against the surface antigen CD20, expressed by B-lymphocytes, is effective in reducing the B-lymphocyte number and has already been successfully used for the treatment of other immunity-connected diseases. In contrast to anti-CD20, the specific anti-EMPD antibody according to the present invention specifically aims at memory cells and plasmablasts.

The antibodies according to the present invention are also preferably used for the preparation of a medicament for the treatment of lymphomas. As mentioned above for autoimmune diseases, anti-CD20 antibodies are also used for fighting lymphomas. However, since most lymphomas are of clonal origin and therefore express antibodies on their surface, apoptosis can be specifically provided in these lymphoma cells with an anti-mIgM, mIgD, mIgG1,2,3,4, mIgA and IgE-antibody according to the present invention.

In each of these therapeutic or prophylactic treatment, the antibodies according to the present invention are applied to the (preferably human) patient in need thereof in an effective dose and administration regime. Doses and administration strategies already used in the present field are also suitable for the present invention with a suitable optimisation according to standard proceedings.

According to another aspect, the present invention can also be applied in vitro for selectively identifying specific B cells and preferably binding the antibody according to the present invention on the surface of the functional cell.

As mentioned above, the present invention also provides an anti-murine memory cell antibody which is obtainable by immunisation of mice, unable to express mIgE, with the MVR of a EMPD-peptide;
(a) removing a preparation of anti-IgE-antibodies from the mice; or
(b) generating hybridomas by using lymph node and/or spleen cells from the mice having anti-IgE-antibodies; and
obtaining anti-IgE-antibodies as antibodies which are specific for murine memory cells from said preparation or hybridomas.

Furthermore, it ia also possible to actively induce the antibodies of the present invention by vaccination with the MVR of EMPD in a suitable vaccine, preferably with a suitable adjuvant and/or a pharmaceutically acceptable diluent or carrier. Preferably, the MVR is delivered as a peptide vaccine with a peptide consisting of 8 to 25 amino acids of the MVR comprising the binding region for an apoptosis inducing antibody according to the present invention.

The present invention is further described in the following examples and in the figure, yet without being restricted thereto.

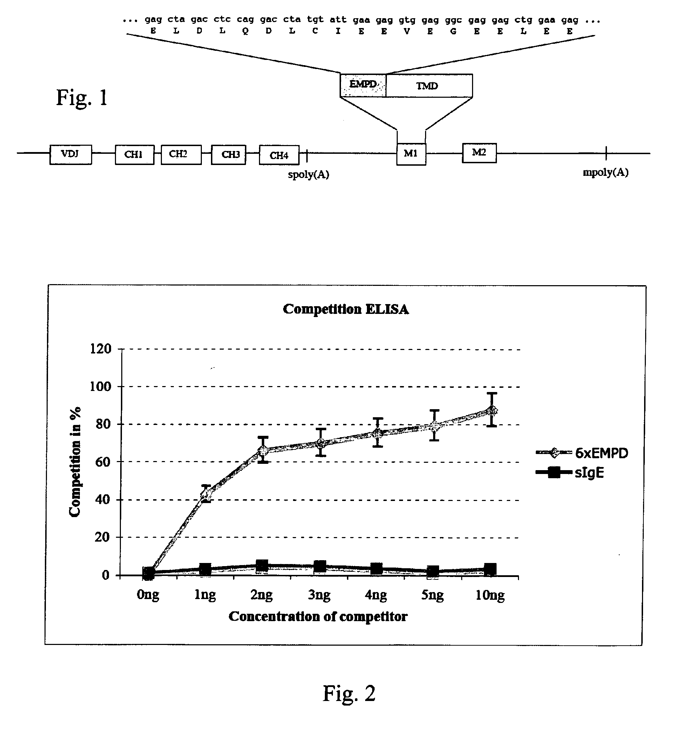

Fig. 1 shows that the membrane form of IgE is coded by the variable domain, 4 constant exons (CH1-CH4), the transmembrane domain M1 and the cytoplasmic domain M2. The 19 amino acids of the extra membrane proximal domain (EMPD), coded by M1, were used as target sequence for the generation of anti-EMPD specific antibodies;

Fig. 2 shows a competition ELISA: 1ng of mAbA9 was pre-incubated with increasing amounts of 6xEMPD antibody. A clear competition effect from 10 to 100 molar 6xEMPD excess was observed. No competition was achieved with serum IgE, thus indicating specificity for the membrane form of IgE;

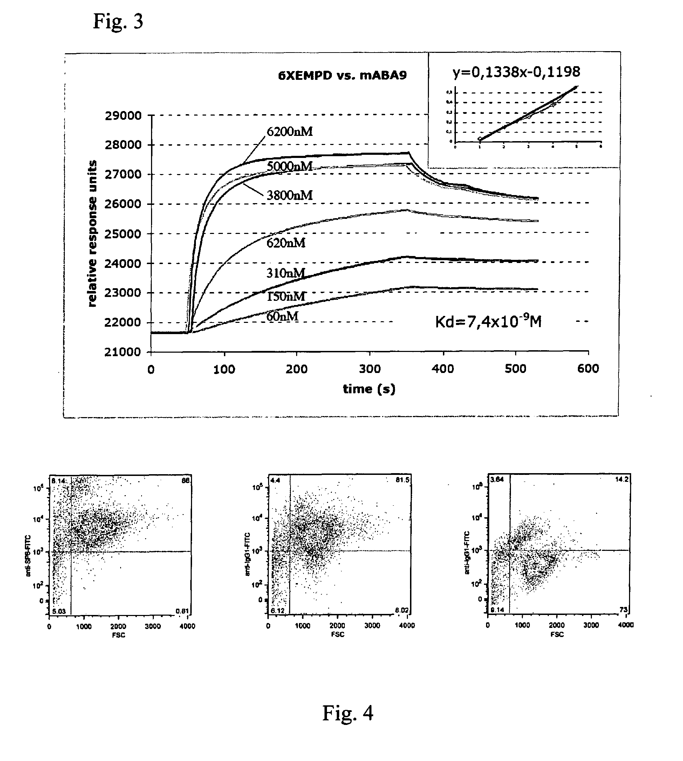

Fig. 3 shows the determination of the binding affinity: SPRA-analysis was performed, by injecting increasing amounts of mAbA9. Relative response units served as data points for the subsequent scatchard blot analysis. The slope of the deduced straight line equation was used to determine the binding affinity constant;

Fig. 4 shows the FACS analysis of mAbA9: Cell line K46, expressing mIgE, was tested for surface recognition of mAbA9. (a) the anti-idiotypic antibody Anti SP6-FITC, recognising the surface expressed mIgE molecule served as positive control. (b) In a sandwich approach, using mAbA9 as primary and anti mouse IgG1-FITC as secondary antibody a comparable shift of the whole population was observed, showing positive recognition of the EMPD-region of mIgE. (c) the secondary anti mouse IgG1-FITC labelled antibody () served as control;

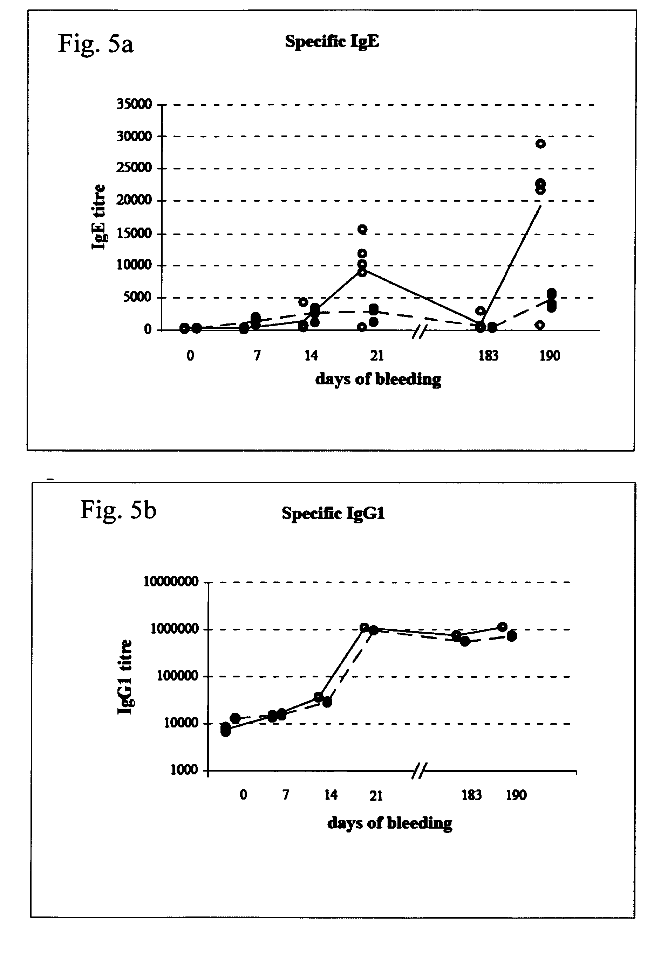

Fig. 5 shows Bet v 1a specific IgE (a) and IgG1 (b) titres after the parallel application strategy: Comparing IgE titres of group 1 (open circles) and group 2 (closed circles) showed a dramatic decrease of Bet v 1a specific IgE titres of group 2 at day 21 and day 190. As expected no difference in the IgG1 specific titre was observed. Each circle (open or closed) represents one individual mouse. Mean values of the different groups are expressed as closed (group 1) ore interrupted (group 2) lines;

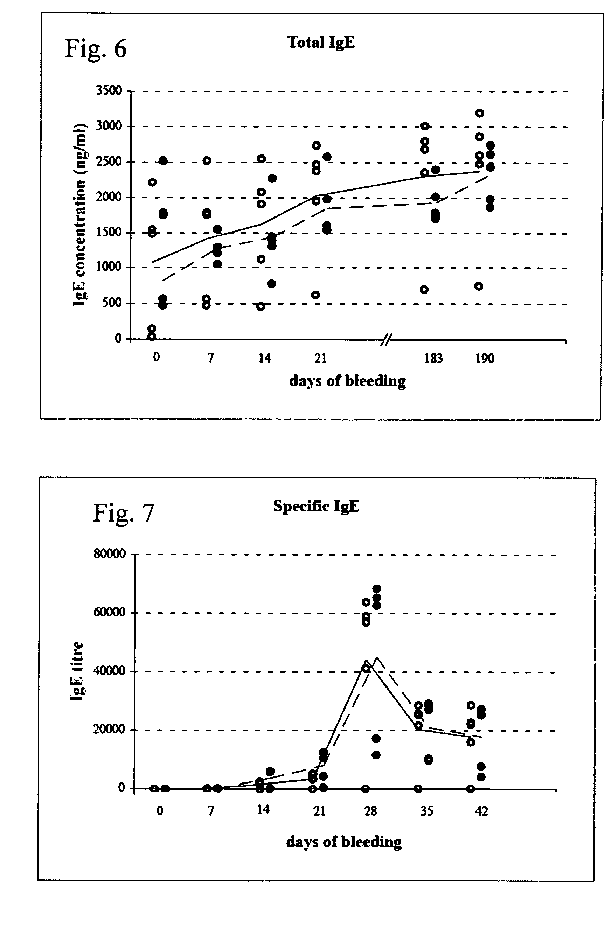

Fig. 6 shows the total IgE levels of group 1 and group 2 following the time course of immunization. No significant decrease of the total IgE level in group 2 (closed circle), compared to group 1 was observed. This shows that total IgE levels cannot be suppressed with mABA9. Each circle (open or closed) represents one individual mouse. Mean values of the different groups are expressed as closed (group 1) ore interrupted (group 2) lines;

Fig. 7 shows that existing Bet v 1a IgE titres are no target of mABA9. Group 3 (open circles) was immunized with Betv1a on day 0, 7 and 14 (control). Bet v 1a IgE titre was measured at time points indicated. Group 4 (closed circles) received mABA9 starting with day 18. As shown very clearly, no difference between the two groups was observed, indicating that already established specific IgE titres cannot be suppressed with mABA9 in a passive immunization approach. Each circle (open or closed) represents one individual mouse. Mean values of the different groups are expressed as closed (group 1) ore interrupted (group 2) lines;

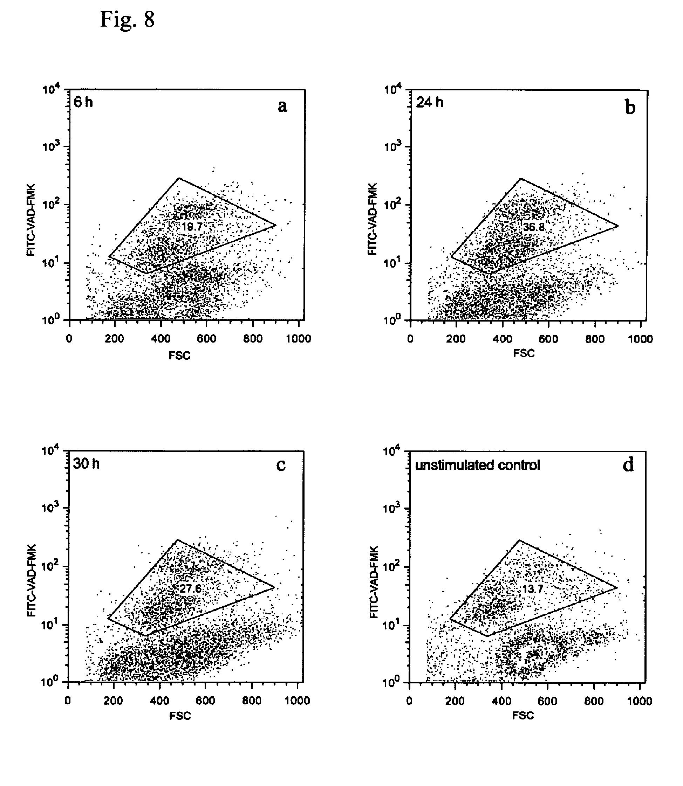

Fig. 8 shows the mAbA9 dependent induction of apoptosis: mIgE expressing cell line K46 was stimulated with mAbA9. Appearance of apoptotic cell populations was observed in a time dependent (ac) experiment using a CaspACE FITC-VAD-FMK apoptosis assay. The maximum of apoptotic cells was observed after 24 hours (b) of incubation. Unstimulated cells after 24 hours served as control (d);

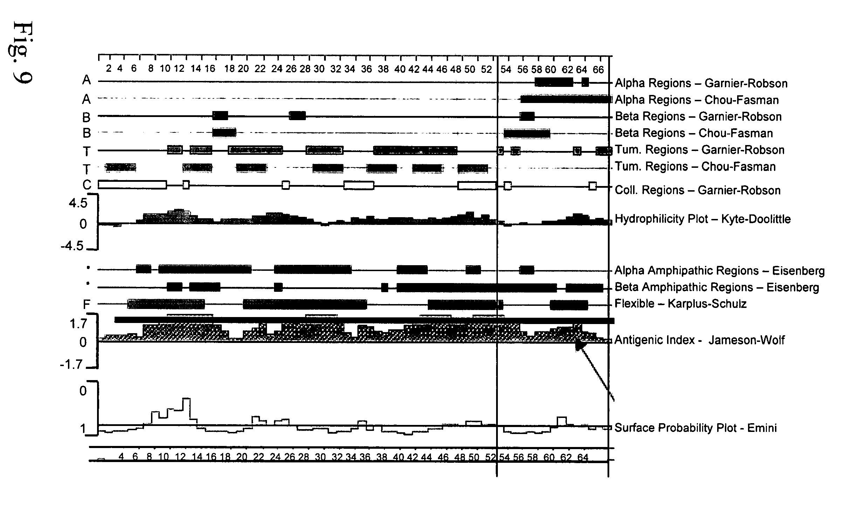

Fig.9 shows an in silicio predicition on the basis of the antigen index showing that the MVR of the EMPD has a significantly lower antigen index as the part of the (long form) being more distant to the transmembrane domain;

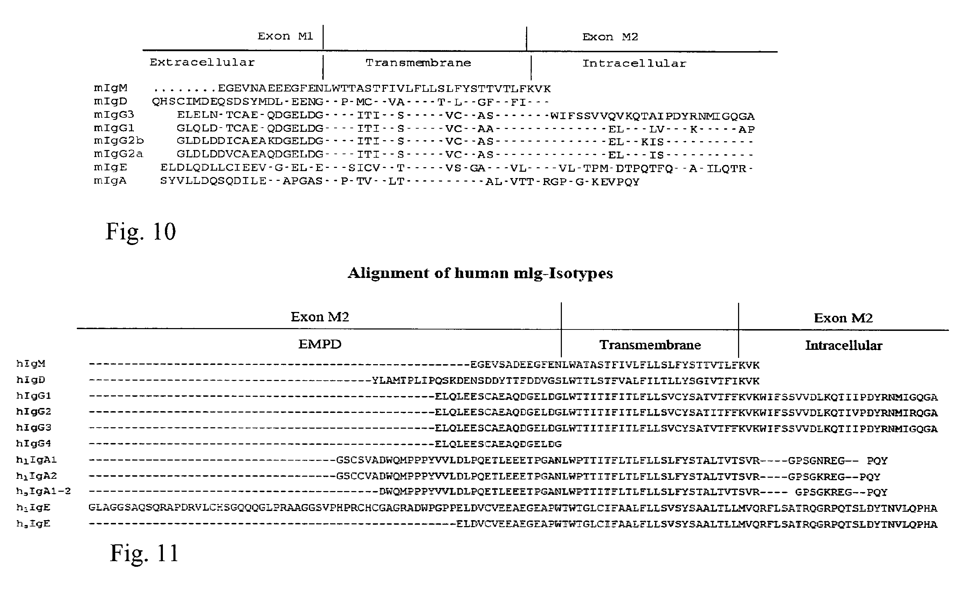

Fig.10 shows relevant amino acid sequences of MVRs of the mIg isotypes in mouse (left block ("Extracellular")); and

Fig.11 shows relevant amino acid sequences of MVRs of the human mIg isotypes (left block ("EMPD")).

### EXAMPLE SECTION:

In the present example section, the working of the present invention is shown in a systemic murine anti-IgE approach, based on the isotype-specific targeting of mIgE expressing cells with a monoclonal antibody raised against the EMPD of mIgE (Figure 1). In the present examples, it is shown that targeting of a mIgE population by passive immunization indeed leads to a dramatic decrease of specific serum IgE levels in vivo and induces apoptosis in vitro. Responsible for the decrease of serum IgE in vivo are tolerance-inducing mechanisms, which stop mIgE B cells in their proliferation and differentiation either through clonal deletion (apoptosis) or through anergy induction. As shown by these results, targeting human mIgE is a reasonable approach with the advantage of inhibiting IgE secretion before production of secreted IgE starts.

### MATERIALS AND METHODS

Immunization and generation of hybridomas:

To circumvent tolerance phenomenon, female ΔM1M2 Balb/c mice (Achatz et al., Int Arch Allergy Immunol 113 (1997), 142-145) unable to express membrane-bound IgE due to the lack of the IgE-transmembrane and IgE-cytoplasmic domains, were immunized with Keyhole Limpet Hemocyanin (KLH) coupled EMPD-peptide of mouse IgE (synthesized by INBIOS, Pozzuoli, ITALY). The KLH-EMPD peptide was precipitated with Alum and injected intranodally (i.n.) (Johansen et al., Eur J Immunol 35 (2000), 568-574). In parallel, a 6 times repeated, recombinant EMPD protein (6xEMPD), expressed as N-terminally [His]₆-tagged protein in E.coli was used for intraperitoneal (i.p.) booster immunization. 0.5 µg of the EMPD-KLH conjugate was injected every two weeks (until day 56) into the left inguinal lymph node of each mouse. After five times, 10µg of 6xEMPD was injected i.p. on day 66 and day 72. Subsequently, the mice were boosted with 10 µg EMPD-KLH i.p. Three times (day 79, 81, and 86).

To generate hybridomas, lymph node and spleen cells of anti-EMPD positive mice were harvested and fused with the mouse B-lymphoma cell line Ag-8. Dulbecco modified Eagle medium (DMEM) (Gibco BRL) was used as wash medium; DMEM supplemented with 25 mM Hepes and 50 % PEG-4000 (Merck) (pH 7.4) as cell fusion medium. Hybridoma cells were disseminated in 16 well cloning plates and selected in HAT-Medium: Optimem (Gibco BRL, Gaithersburg, USA) supplemented with 5 % fetal calf serum (FCS, Gibco BRL), 1x Pen/Strep, 50 U/ml IL-6 (Roche), 13.6 µg/ml Hypoxanthine (Sigma), 3.88 µg/ml Thymidine (Sigma-Aldrich), and 0.175 µg/ml Aminopterine (Sigma). Cells were incubated at 37°C, 7% CO₂. Culture supernatants were tested for anti-EMPD antibodies by ELISA and positive lines cloned by limiting dilution to obtain specific monoclonal hybridomas.

### Purification of the monoclonal antibody:

Anti-EMPD-specific hybridomas were cultivated in tissue culture flasks (Greiner) for 14 days and finally centrifuged at 13 000 g, at 4°C for 30 min. To adjust the pH, 1/10 vol of 1 M Tris/Cl pH 8.0 was added to the supernatant before precipitation with saturated ammonium sulphate (45 % v/v). The solution was stirred at 4°C for three hours. After centrifugation at 15000 g, 4°C for 30 min., the supernatant was decanted and the pellet was dissolved in ice-cold PBS. For subsequent purification with an Äkta prime device the dissolved antibody solution was dialysed against 20 mM sodium phosphate pH 7.1 and filtered through a 0.45 µm membrane.

For the chromatographic purification all buffers and solutions were degased. First, the device was rinsed with elution buffer (0.1 M Glycine/HCL pH 2.7) followed by binding buffer (20 mM sodium phosphate pH 7.1). The HiTrap^{™} column was washed with H₂O and binding buffer was integrated into the tube system. The sample was applied and the column was washed with 5 - 10 column volumes of binding buffer until no material appeared in the effluent any more. Elution was carried out with elution buffer and fractionation in 500 µl samples. For pH-neutralization 100 µl 1 M Tris/Cl pH 8.1 was added to each fraction. The fractions were tested for their IgG1 content under reducing and non-reducing conditions in Coomassie^{™}-staining and Western Blot with AP-labeled goat anti-mouse IgG1. Positive fractions were pooled and dialysed against PBS with 0.05 % NaN₃. The protein concentration was measured with BCA-assay kit (Pierce, Rockford,USA).

### Sequence analysis:

RNA of the hybridoma cells was isolated with RNeasy kit (Promega), followed by cDNA synthesis with first strand reaction beads and oligo(dT)-primer. DNA amplification was performed with a mix of V_{H} primers (V_{Ha}: [5'-gaggttcagctgcagcag (ct) c-3' ] ; V_{Hb}: [5'-gaggtgcagctggtgga(ag)tc-3']) and a constant reverse primer for γ1 heavy chain (C_{H}2γ1: [5'-ttaggagtcagagtaatggtgagcacatcc-3']), and a mix of V_{L} primers (V_{K}1: [5'-gatgttttgatgacccaaactcca-3']; V_{K}4: [5'-caaattgttctcacccagtctcca-3']; V_{K}10: [5'- gatatccagatgacacagactaca-3']; V_{K}24: [5'-gatattgtgatgacgcaggctgca-3']) and a constant κ light chain reverse primer ([5'-gatggatacagttggtgc-3']).

The γ1 chains were cloned into pCR-scriptTM Amp vector (Qiagen). Analysis of the sequence was performed with the ABI-PRISM® sequencing kit of Applied Biosystems using M13 forward [5'-gtaaaacgacggccagt-3'] and reverse [5'-ggaaacagctatgaccatg-3'] primers.

### Determination of the binding affinity by surface plasmon resonance (BIACORE X):

Recombinant His-tagged 6xEMPD (250µg/ml in PBS) was diluted 1:1 in 10 mM NaAcetate pH 4 and coupled to CM5 chips according to the manufacturer's instructions. Approximately 1700 RUs rec. 6xEMPD-His were coupled to flow cell 1. Empty flow cell 2 served as reference. mAbA9 was injected at different concentrations (12 nM - 1,2 µM) in HBS-EP buffer (Biacore) and the surface plasmon resonance was recorded. Data were analysed with BIAevaluation software (Biacore).

### ELISA:

### Competition ELISA:

Nunc-96-well^{™} plates were coated with 50ng 6xEMPD per well. 1ng mAbA9 was preincubated with increasing amounts of 6xEMPD peptide from 1 to 10ng in 50µl each. After 2 h, the pre-incubated antibody-peptide complex was added to the 6xEMPD coated plate followed by incubation for 2 h at RT. As secondary antibody AP-labelled goat anti-mouse IgG1 (1:3000 in PBS) was incubated at RT for one hour. After development with AP substrate, the absorption was measured at 405 - 492 nm. Data were expressed as % competition against 6xEMPD concentration.

### Total IgE ELISA:

Coating was done with 500 ng/100 µl per well of anti-IgE 84.1C in PBS o.n. After blocking with PBS containing 1% BSA (1 h, 37 °C) plates were washed with PBS and mice sera were added in dilutions 1:20, 1:60, and 1:180 in PBS/0,1% BSA at RT for 2 h. As standard, purified mouse IgE was used. As detection antibody AP-labelled rat anti-mouse IgE (EM95/3), diluted at 1µg/ml in PBS/0,1% BSA was used. The absorption was measured at 405 - 492 nm.

### Specific IgE ELISA:

For antigen-specific ELISA measurements Nunc-96-well^{™} plates were coated with 500 ng recombinant Bet v1 a/well. Sera were diluted 1:10, 1:30 and 1:90 in PBS containing 1%BSA added to the plate and incubated at 4°C o.n. For detection AP-labelled rat anti-mouse IgE (EM95/3) was added at a concentration of 1µg/ml in PBS/0,1% BSA and plates were incubated at RT for 2 hours. Absorption measurement was read out at 405 - 492 nm. Titres were expressed as the reciprocal serial dilution, at which a half-maximal OD value was measured.

### FACS analysis:

K46 mIgE+ cells were centrifuged at 260 g for 5 min. The pellet was washed twice with FACS buffer. Cells were incubated with 500ng of purified anti-EMPD antibody and incubated on ice for 30 min. As secondary antibody we used a goat anti-mouse IgG1-FITC conjugate.

### RBL-Assay:

Rat basophilic lymphocytes (RBLs) were plated to 4 x 10⁴ cells/well in a volume of 100 µl RPMI - RBL medium in flat-bottom wells tissue culture plate and incubated overnight at 37°C and 7,5 % CO₂. On the next day 50µl supernatant was removed, and serum dilutions ranging from 1:20 to 1:320 were added in prewarmed RPMI. Plates were further incubated for 2 h, at 37°C followed by two washes with 200 µl tyrode/BSA wash buffer. For stimulation 5 µg Bet v 1a, anti IgE antibody EM95/3, 84-1C or mAbA9 were added at different concentrations. Cells are then incubated again for 30 min at 37°C. For a 100 % release of β-hexosaminidase, cells were lysed by adding 1 % triton X-100. Plates were then centrifuged for 5 min, 260 x g at RT. Afterwards 50 µl supernatant was transferred to a new 96-well. 50 µl assay solution (0,1M citric acid pH 4,5, 80µl 10mM 4-Methylumbelliferyl N-Acetyl-β-DGlucosaminide) was added and the plates were incubated at 37°C and 7,5 % CO₂ for 1 h. The reaction was stopped by addition of 100µl glycine pH 10,7. The fluorescence (SpectraFlour, TECAN, Austria) was measured at the bottom of the plate (excitation 360 nm; emission 465 nm). Measured values were converted to % of total release obtained by triton lysis.

### CaspACE FITC-VAD-FMK apoptosis assay:

mIgE-positive K46 cells were incubated with mAbA9 at a concentration of 0.3µg/ml in a total volume of 1ml in RPMI/7.5% FCS in flat bottom 48-well plates (Greiner). After 6, 24, 30, 48, 54 and 72 h the percentage of apoptotic cells was measured using the CaspACE FITC-VADFMK (Promega) apoptosis assay, according to manufacturer's instructions.

### RESULTS:

### Selection of mIgE-EMPD specific Hybridomas: Immunization/Fusion /Purification:

5 ΔM1M2 mice were pre-immunized intranodally (i.n.) with 500 ng of the KLH coupled EMPD-peptide (KLH-ELDLQDLCIEEVEGEELEE) of mouse mIgE and boosted i.p. with purified His6-tag recombinant, 6 times repeated EMPD protein (6xEMPD; HHHHHH(ELDLQDLCIEEVEGEELEE)₆), cloned and expressed in vector pHIS2 parallel. The increase in specific anti-EMPD titre was measured with ELISA. At day 77 and 85 two of the five mice showed significantly high specific IgG1 titres. The spleens and lymph nodes of these two mice were used for hybridoma fusion. After several limiting dilution steps, supernatants were tested again for anti-EMPD secreting antibodies. Finally, ten single hybridomas were identified as anti-EMPD secreting clones of IgG1 isotype. One positive clone (A9) was selected for production and purification of the corresponding mAb (mAbA9). Ammonium sulfate precipitated hybridoma culture supernatant was dialysed against Äkta prime loading buffer and loaded onto a HiTrapTM Protein G HP column. Elution was carried out with glycine/HCl pH 2.7 and fractions were collected and analysed by 7.5 % SDS-PAGE. Positive fractions were pooled, dialysed against PBS, concentrated and the protein amount determined. 150 ml hybridoma cell culture supernatant yielded 2 mg of purified monoclonal antibody (mAbA9).

### Cloning and sequencing of the anti-EMPD specific antibody mAbA9:

Total mRNA was isolated from hybridoma cells. After first strand cDNA synthesis using an oligo(dT)-primer, the heavy- and light-chains were amplified with primer mixes for VH and VL variable region and specific constant region primers. Amplicons were cloned into the pCR-ScriptTM Amp vector and both strains sequenced. Blast search identified the variable heavy region of anti-EMPD γ1-chain as member of the V11 germline gene family and the variable region of the K light chain as member of the kj4 germline gene family. Variable heavy- and light-chain sequences of the mAbA9 antibody were submitted to GenBank (accession numbers: EF156450 for the VH, and EF156451 for VL).

### Specificity of the anti-EMPD mAbA9 in Competition-ELISA:

To test the specificity of mAbA9 for 6xEMPD competition-ELISAs were performed. Competition was achieved by pre-incubation of 1ng mAbA9 with increasing amounts of purified recombinant 6xEMPD peptide. With 10 fold molar excess of 6xEMPD peptide 50% competition was measured, while 95% inhibition could be obtained with 100 molar excess. No competition was observed with soluble mouse IgE, indicating specificity of mAbA9 for mIgE (Figure 2).

### mAbA9 binds the extra membrane proximal domain with high affinity:

The association between the recombinantly expressed His-tagged 6xEMPD peptide and mAbA9 was tested by surface plasmon resonance analysis (SPRA) using a Biacore X device. 1700 resonance units (RUs) of recombinant His-tagged 6xEMPD were coupled to flow cell 1 of a CM5 chip. The empty flow cell 2 served as reference. The purified mAbA9 was injected at different concentrations (12 nM - 1,2 µM) and the surface plasmon resonance was recorded. The KD of 7.5 x10⁻⁹ M determined using scatchard blot analysis from the respective curves (Figure 3) shows a high affinity of mAbA9 for the substrate.

### mAbA9 recognises native mIgE expressed on the mouse lymphoma cell line K46:

To demonstrate the capacity of the anti-EMPD mAbA9 to bind surface expressed mIgE antibodies, the mIgE-expressing cell line JW813/4 was incubated with the unlabelled purified anti-EMPD antibody. For detection a FITC-labelled goat anti-mouse IgG1 antibody was used. A shift of the whole population could be clearly detected, reflecting the capacity of the anti-EMPD antibody to recognize surface expressed mIgE (Figure 4).

### mAbA9 does not interact with FcεRI bound soluble IgE:

RBL cells cultivated as described were incubated with serially diluted serum samples of Betv1a immunized mice. Total cell lysis with triton X-100 led to a complete release of β-hexosaminidase and served as control (100%). Addition of Bet v1a antigen led to a release of 7,6%, indicating the specificity of the IgE antibodies for Bet v1a. Rat anti mouse IgE antibody EM95-3 and 84-1C induced a release of 27, 8% and 17%, respectively. As expected, incubation of receptor bound IgE with two concentrations of mAbA9 do not induced any relevant release of β-hexosaminidase (1,8% and 2%). These results show that mAbA9 is a non anaphylactic anti IgE antibody unable to cross-link receptor bound IgE molecules (Table 1):

**Table 1:**

| Stimulation | | | | | | | |
|---|---|---|---|---|---|---|---|
| | Triton X-100 | Bet v1a (5µg/100µl) | mAbA9 (5µg/100µl) | mAb A9 (100µg/100µl) | Em95/3 (7µg/100µl) | 84-1C (7µg/100µl) | Negative control |
| %Release | 100 | 7,6 | 1,8 | 2 | 27,8 | 17 | 1,3 |

To test that mAbA9 has no anaphylactic capacity, RBL cells were incubated with serum of Betv1a immunized mice and subsequently stimulated with Bet v 1a, mABA9 and two different anti-IgE antibodies (EM95/3 and 84-1C). The RBL assay clearly shows that mAbA9 does not lead to a degranulation of RBL cells.

Passive administration of mAbA9 drastically decrease specific serum IgE levels:
Mice of control group 1 were immunized with the allergen Bet v 1a on day 0, 7, 14, and 183 (Table 2).

**Table 2:**

| | Group 1 | | Group 2 | | | Group 3 | | Group 4 | |
|---|---|---|---|---|---|---|---|---|---|
| | Betv1 | mAbA9 | Betv1 | mAbA9 | | Betv1 | mAbA9 | Betv1 | mAbA9 |
| d0 | + | | + | | d0 | + | | + | |
| d3 | | | | + | d7 | + | | + | |
| d6 | | | | + | d14 | + | | + | |
| d7 | + | | + | | d18 | | | | + |
| d9 | | | | + | d21 | | | | + |
| d12 | | | | + | d24 | | | | + |
| d14 | + | | + | | d27 | | | | + |
| d15 | | | | + | d30 | | | | + |
| d183 | + | | + | | d33 | | | | + |
| | | | | | d36 | | | | + |

Immunization scheme of the 4 mouse groups receiving either exclusively recombinant Bet v 1a, or Bet v 1a in combination with mAbA9.

Group 2 received the antigen as described for group 1, but additionally received monoclonal anti-EMPD antibody every three days from day 3 onwards according to the immunization scheme shown in Tab. 2. The "3-day scheme" reflects the half life of IgG1 in the serum of mice. The specific Bet v 1a titre in the control group continually increased during the time course of immunization, and reached a first peak at day 21. It is known that specific IgE titres decreased rapidly in this mouse model. However, a clear memory response to Bet v 1a was detectable after a booster immunization (day 183) at day 190. In contrast, parallel application of mAbA9 strongly repressed Bet v1a specific IgE titres. At day 21, the allergen-specific IgE titre was decreased by 67%. Although, after day 15, group 2 did not receive mAbA9 any more, a strong suppression of allergen-specific IgE was still present at day 190 as reflected by the measured 74% decrease of the titre compared to the control group (Figure 5a). These data are conceivable with a missing memory response due to the depletion of IgE-specific Bet v 1a memory B cells.

This is corroborated by the fact that the Bet v 1a specific IgG1 antibody response was not affected by passive immunisation with the anti-mIgE mAbA9 antibody. No differences on the IgG1 titres between control and treated group could be observed during the time course of immunization (Figure 5b).

### Total IgE levels are not affected by the passive immunization:

It was next asked if the total IgE level was affected in mAbA9 immunized mice. Groups 1 and 2 mice (Table 2) were tested for their total IgE serum levels. During the course of immunisation, total IgE levels weakly increased from 1µg/ml up to 2,5 µg/ml, without any significant difference between the investigated groups (Figure 6). This shows that mAbA9 had no effect on total IgE levels. Accordingly, IgE without specificity form Bet v 1a is produced by pre-existing long lived plasma cells.

### Existing specific IgE Bet v1a titres are not influenced by mAbA9 treatment:

To test if passive mAbA9 immunisation led to a decrease of already established Bet v1-specific IgE responses the following experiment was performed: Control group 3 (Table 2) was immunized with Bet v 1a on day 0, 7 and 14. Group 4 was pre-immunized with Bet v 1a as described for group 3, but received mAbA9 seven times, starting with day 18. Bet v 1-specific IgE serum titres were analysed by ELISA at day 0, 7, 14, 21, 28, 35, and 42, and continually increased in both groups during the time course of immunization. After reaching the highest concentration at day 28 they rapidly decreased without significant difference between the two groups (Figure 7).

### mAbA9 induces apoptosis in mIgE expressing cell lines:

It was tested if receptor mediated stimulation can induce apoptosis of mIgE-expressing B cells in vitro. mIgE transfected K46 mouse lymphoma cells were stimulated with mAbA9. Subsequent FACS analysis showed a time dependent increase of an apoptotic cell population compared to unstimulated cells (Figure 8). After 6 hours an apoptotic cell population of 19% could be detected. After 24 hours the maximum of 36% apoptotic cells was reached followed by a decrease of apoptotic cells to 27% after 30 hours. As control, unstimulated cells after 24 hours were used. This result clearly demonstrates that mAbA9 induces apoptosis in a receptor mediated process.

### DISCUSSION

Several strategies to treat IgE-mediated allergic diseases by down-regulating IgE levels were developed in the recent past. Although different hypotheses explaining the effectiveness of therapy based on isotype-specific anti-IgE antibodies were published, a general accepted concept cannot be presented so far. The basic idea of this therapy is to use anti IgE antibodies with unique binding properties to control serum IgE levels. The basic requirements to be fulfilled by such therapeutic antibodies are: I) high affinity for IgE; II) the ability to neutralize free IgE; III) lack of affinity for IgE bound to effector cell either through the high affinity receptor FcER1 or the low affinity receptor FcεRII (CD23) to avoid degranulation. Therapeutic treatment with such non-anaphylactogenic anti-IgE mAb's leads primarily to a decrease of serum IgE levels in allergic individuals, mainly due to the depletion of free serum IgE. The mechanisms underlying the clinical efficacy of anti-IgE mAb's, which bind free IgE at the same site as the FccR1 high affinity receptor, are largely unknown, but could involve inhibition of mast cell and basophil activation through a combination of reduced free IgE levels and IgE receptor down-regulation. However, the effects of these antibodies on memory B cells which express membrane-bound IgE as integral part of the B cell receptor (BCR) are unknown. Therefore, mAb's able to specifically target membrane-bound IgE which, in the context of the BCR is indispensable for the production and quality of secreted IgE, were developed in the course of the present invention as a practical application example for this invention. The ability to block IgE synthesis in vivo was tested. mIgE is a spicing variant of soluble IgE which contains, beside a transmembrane region and a cytoplasmic tail, an isotype-specific 19-amino-acid long extracellular membrane proximal domain (EMPD, Figure 1). A recombinantly produced EMPD multimeric fusion protein to immunize ΔM1M2 mice (Achatz et al., 1997) was produced. This special mouse strain was used to avoid tolerance observed in mice expressing a functional mIgE bearing BCR. EMPD protein was administered through the intralymphatic route followed by intraperitoneal booster immunization to enhance the immune response.

Using this procedure a high affinity (Figure 3) mouse monoclonal anti-mouse-EMPD antibody of γ1-isotype termed mAbA9 was isolated. Specificity for the 19 amino acids of the IgE-EMPD region was demonstrated by ELISA, inhibition ELISA and by the lack of binding to soluble IgE as well as to antibodies of other isotypes (Figure 2). FACS analysis of the mIgE-expressing K46 cell line was used to demonstrate the ability of the mAbA9 to recognize the native conformation of mIgE (Figure 4). However, to fulfil the criteria for therapeutic use, anti-IgE mAb's should be non anaphylactic. Table 1 demonstrates that even high concentrations mAbA9 lack the capability to induce degranulation of RLB cells. At this stage mAbA9 fulfilled all criteria required to be a promising lead compound for passive immunization experiments.

In a prophylactic study mAbA9 was passively administrated to mice in parallel with Bet v 1a as sensitizing allergen. Compared with the control group only treated with Bet v 1a a drastic reduction in the production of Bet v la-specific IgE antibodies at day 21 (-64%) with a long lasting effect on the development of a Bet v 1-specific IgE-memory cell population was observed. In fact, challenge of both mice groups with Bet v 1a at day 183 quickly restored the Bet v1-specific IgE titer levels at day 190, whereas the mAbA9 treated group still showed a marked reduction of the titres (74%) in spite of the fact that the administration of the anti-mIgE antibody was stopped at day 15 (Figure 5a). With this experiment it is evident that the passive immunisation with mAbA9 strongly suppress the development of an allergen-specific IgE memory, whereas antibody responses of other isotypes are not influences as exemplified for the Bet v 1-specific IgG1 response (Figure 5b). Therefore, mAbA9 induces strong apoptosis in vitro if added to mIgE-expressing K46 cells. The extremely low number of mIgE-expressing B cells present in vivo hampers a direct, satisfactory FACS analysis of this population, which would represent a suitable method to show apoptosis events. Interestingly the total serum IgE levels are not influenced by them AbA9 treatment (Figure 6). It follows that total serum IgE results from pre-existing IgE-producing long living plasma cells which are not a target for mAbA9 because the do not express mIgE on their surface, but reside in survival niches of the bone marrow. However, since new developing IgE secreting plasma cells go through mIgE-expressing B cell stages during differentiation, and their generation is abrogated by anti-mIgE treatment, the existing plasma cells will die off in several weeks to several months, and thus the production of IgE will gradually abate in similar periods.

This is in line with the fact that a curative passive immunization with mAbA9 in mice with an established Bet v 1a-specific IgE response fails to reduce the titres of allergen-specific IgE (Figure 7). In this experiment mice were sensitized with Bet v 1a as in the prophylactic experiment (see treatment schedule, Table 2) and then passively immunized with mAbA9 starting with day 18 after the first Bet v 1a application at three days intervals until day 36.

Summarizing, passively administered mAbA9 does not affect already established total IgE populations, nor already established specific IgEs against Bet v 1a, but clearly shows an prophylactic impact on developing specific IgE titres. This prophylactic impact of mAbA9 could be based on the following molecular mechanisms: the B cell binds the antigen via its receptor, which transduces the first signal into the cell via activation of tyrosine kinases, such as syk and lyn (reviewed in Geisberger et al, Immunology 110 (2003), 401-410). The receptor-antigen complex is then internalized, the antigen processed, and displayed as peptide MHC II complex on the B cell surface. Antigen binding to the B cell also stimulates expression of the co-stimulatory molecules B7-1 and B7-2. If T cells exist with receptors that can recognize these co-receptor-peptide-MHC complexes, then the ensuing T cell B cell interaction provides the second signals in the form of a CD40-ligand CD40 interaction and the release of stimulatory cytokines such as IL4 and IL5 or IFN-γ. In this framework, antigen stimulation of B cells in the absence of T cell help leads to tolerance, while stimulation in the presence of antigen specific T cell help leads to proliferation and differentiation. In other words, stimulation of B cells via the antigen receptor without appropriate T-cell help normally leads to apoptosis.

These data can be adapted for the induction of tolerance and/or anergy of a class-switched mIgE bearing B cell population by using anti IgE antibodies. For example, the following scenarios for the functioning of a humanised anti mIgE antibody, which by the immune system is considered as an auto antigen, leading to no recruitment of T cells, are immediately at hand from the present examples. First, as shown by self reacting immature B cells, cross linkage of the mIgE receptor without further T cell support should directly induce apoptosis. Second, as normally shown during the induction of peripheral tolerance of mature B cells in answer to monovalent (self) antigen, receptor blockage of mIgE should result in an anergic state of the mIgE population. In contrast to normal mature B cells, which have a half life of 4 to 5 weeks, anergic B cells were found to last for only 3 to 4 days. Nevertheless the fate of these anergic B cells finally turns out to be cell death.

Indeed, mAbA9 could induce apoptosis in a K46 cell line, expressing mIgE (Figure 8). Therefore the decrease of specific serum IgE results in an active elimination of the mIgE bearing B cells. Targeting mIgE bearing B cells with anti-mIgE specific antibodies is therefore a promising therapeutic alternative in the future. The advantage of this therapeutic approach is the next step in the generation of anti IgE therapy, with the advantage of inhibiting IgE secretion before specific secreted IgE production starts.

These results are even more surprising, if a computer prediciton for the antigenic index ("Antigenic index - Jameson-Wolf") is regarded (see Fig. 9; arrow). According to this computer prediciton, suitable epitopes should have been found in the region of EMPD of mIgE being more distant and part of the long form of this variant. Only in this more distant regions, three promising regions have been identified whereas no promising epitope can be detected in the MVR (corresponding to the short form in mIgE).

The MVR as used in the present invention relates to the region extending from the membrane to the extracellular space, i.e. the amino acids immediately adjacent to the transmembrane domain (usually beginning with an LW or TW dipeptide in humans). For example in the murine system, relevant amino acid sequences of the MVR of the mIg isotypes in mouse and humans are depicted in Figs. 10 and 11 in the left block ("Extracellular"; "EMPD"). The present invention covers all homologues of MVR in mammals, i.e. the corresponding sequences to the sequences of Figs. 10 and 11 in the different mammalian species. Specifically preferred are, of course, the human sequences and the application of the present invention in the human system (in human patients). For example with respect to mIgE, the specifically preferred MVR is defined as follows (regarding the special circumstance of having a long and a short isoform): 
hₗ-E: GLAGGSAQSQRAPDRVLCHSGQQQGLPRAAGGSVPHPRCHCGAGRADWPGPPELD---VCVEEAEGEAP

hₛ-E: --ELD---VCVEEAEGEAP

mₛ-E: --ELDLQDLCIEEVEGEELEE
 (h₁-E: human long epsilon EMPD (MVR: C-terminal 16 amino acids); hₛ human long epsilon EMPD (MVR: C-terminal 14 amino acids); mₛ-E: murine short epsilon EMPD (MVR: C-terminal 19 amino acids)).

Preferred (up to maximal 20 amino acid resiues in N-terminal direction from the "membrane border" (transmembrane section usually begins with a LW or TW dipeptide) human MVR regions from other isotypes are the following (see also: Fig. 11):

| | |
|---|---|
| hIgM: | EGEVSADEEGFEN |
| hIgD: | (YL)AMTPLIPQSKDENSDDYTTFDDVGS |
| hIgG1: | ELQLEESCAEAQDGELDG |
| hIgG2: | ELQLEESCAEAQDGELDG |
| hIgG3: | ELQLEESCAEAQDGELDG |
| hIgG4: | ELQLEESCAEAQDGELDG |
| h₁IgA1 : | ([GS]CSVAD)WQMPPPYVVLDLPQETLEEETPGAN |
| h₁IgA2 : | ([GS]CCVAD)WQMPPPYVVLDLPQETLEEETPGAN |
| hₛIgA1-2: | (D)WQMPPPYVVLDLPQETLEEETPGAN |
| h₁IgE: | ([GLAGGSAQSQRAPDRVLCHSGQQQGLPRAAGGSVPHPRCHCG]AGRAD)WP GPPELDVCVEEAEGEAPW |
| hₛIgE: | ELDVCVEEAEGEAPW |

Antibodies according to the present invention, especially monoclonal, polyclonal or synthetic antibodies (including antibody fragments), binding to these sequences can also be made according to standard proceedings well available to the skilled man in the art. Especially antibodies having a binding affinity to the mIg of at least 10⁻⁸ M can easily be obtained by optimising an initial set of antibodies with the desired binding specificity by standard proceedings.

## Claims

1. Anti-mIg-antibody **characterised in that** it binds specifically to the membrane-vicinal region (MVR) of the extracellular membrane-proximal domain (EMPD) of membrane bound human immunoglobulins (mIgs).

2. Anti-mIg-antibody according to claim 1 **characterised in that** it is a humanised antibody.

3. Anti-mIg-antibody according to claim 1 **characterised in that** it is a murine antibody.

4. Anti-mIg-antibody according to any one of claims 1 to 3 **characterised in that** it has an affinity to the mIg of at least 10⁻⁸ M, especially of at least 10⁻⁹ M.

5. Anti-mIg-antibody according to any one of claims 1 to 3 **characterised in that** it specifically binds to an mIgA, mIgG, mIgE or mIgM, especially to an mIgE.

6. Use of an antibody according to any one of claims 1 to 5 for the preparation of a medicament causing the apoptosis of B cells.

7. Use of an antibody according to any one of claims 1 to 5 for the preparation of a medicament for a specific immunotherapy, especially for preventing allergic reactions.

8. Use of an antibody according to any one of claims 1 to 5 for the preparation of a medicament for prevention of sensibilisation side effects of a anti-acidic treatment, especially for gastric or duodenal ulcer or reflux.

9. Use of an antibody according to any one of claims 1 to 5 for the preparation of a medicament for the treatment of autoimmune diseases.

10. Use of an antibody according to any one of claims 1 to 5 for the preparation of a medicament for the treatment of lymphomas.

11. Anti-murine Memory cell antibody obtainable by immunisation of mice, unable to express mIgE, with the MVR of a EMPD-peptide;
(a) removing a preparation of anti-IgE-antibodies from the mice; or
(b) generating hybridomas by using lymph node and/or spleen cells from the mice having anti-IgE-antibodies; and
obtaining anti-IgE-antibodies as antibodies which are specific for murine memory cells from said preparation or hybridomas.


SEQUENCE LISTING

<110>  BIOMAY AG

<120>  Apoptosis inducing antibodies

<130>  R 49646

<140>  EP 07450062.0
<141>  2007-03-23

<160>  22

<170>  PatentIn version 3.4

<210>  1
<211>  21
<212>  DNA
<213>  Artificial

<220>
<223>  Primer

<400>  1
gaggttcagc tgcagcagct c                                                21

<210>  2
<211>  21
<212>  DNA
<213>  Artificial

<220>
<223>  Primer

<400>  2
gaggtgcagc tggtggaagt c                                                21

<210>  3
<211>  30
<212>  DNA
<213>  Artificial

<220>
<223>  Primer

<400>  3
ttaggagtca gagtaatggt gagcacatcc                                       30

<210>  4
<211>  24
<212>  DNA
<213>  Artificial

<220>
<223>  Primer

<400>  4
gatgttttga tgacccaaac tcca                                             24

<210>  5
<211>  24
<212>  DNA
<213>  Artificial

<220>
<223>  Primer

<400>  5
caaattgttc tcacccagtc tcca                                              24


<210>  6
<211>  24
<212>  DNA
<213>  Artificial

<220>
<223>  Primer

<400>  6
gatatccaga tgacacagac taca                                              24


<210>  7
<211>  24
<212>  DNA
<213>  Artificial

<220>
<223>  Primer

<400>  7
gatattgtga tgacgcaggc tgca                                              24


<210>  8
<211>  18
<212>  DNA
<213>  Artificial

<220>
<223>  Primer

<400>  8
gatggataca gttggtgc                                                     18


<210>  9
<211>  17
<212>  DNA
<213>  Artificial

<220>
<223>  Primer

<400>  9
gtaaaacgac ggccagt                                                      17


<210>  10
<211>  19
<212>  DNA
<213>  Artificial

<220>
<223>  Primer

<400>  10
ggaaacagct atgaccatg                                                    19


<210>  11
<211>  19
<212>  PRT
<213>  Artificial

<220>
<223>  EMPD peptide

<400>  11

Glu Leu Asp Leu Gln Asp Leu Cys Ile Glu Glu Val Glu Gly Glu Glu
1               5                   10                  15

Leu Glu Glu

<210>  12
<211>  120
<212>  PRT
<213>  Artificial

<220>
<223>  EMPD peptide

<400>  12

His His His His His His Glu Leu Asp Leu Gln Asp Leu Cys Ile Glu
1               5                   10                  15

Glu Val Glu Gly Glu Glu Leu Glu Glu Glu Leu Asp Leu Gln Asp Leu
            20                  25                  30

Cys Ile Glu Glu Val Glu Gly Glu Glu Leu Glu Glu Glu Leu Asp Leu
        35                  40                  45

Gln Asp Leu Cys Ile Glu Glu Val Glu Gly Glu Glu Leu Glu Glu Glu
    50                  55                  60

Leu Asp Leu Gln Asp Leu Cys Ile Glu Glu Val Glu Gly Glu Glu Leu
65                  70                  75                  80

Glu Glu Glu Leu Asp Leu Gln Asp Leu Cys Ile Glu Glu Val Glu Gly
                85                  90                  95

Glu Glu Leu Glu Glu Glu Leu Asp Leu Gln Asp Leu Cys Ile Glu Glu
            100                 105                 110

Val Glu Gly Glu Glu Leu Glu Glu
        115                 120

<210>  13
<211>  66
<212>  PRT
<213>  Artificial

<220>
<223>  membrane-vicinal region (MVR) of the extracellular
       membrane-proximal domain (EMPD)

<400>  13

Gly Leu Ala Gly Gly Ser Ala Gln Ser Gln Arg Ala Pro Asp Arg Val
1               5                   10                  15

Leu Cys His Ser Gly Gln Gln Gln Gly Leu Pro Arg Ala Ala Gly Gly
            20                  25                  30

Ser Val Pro His Pro Arg Cys His Cys Gly Ala Gly Arg Ala Asp Trp
        35                  40                  45

Pro Gly Pro Pro Glu Leu Asp Val Cys Val Glu Glu Ala Glu Gly Glu
    50                  55                  60

Ala Pro
65

<210>  14
<211>  14
<212>  PRT
<213>  Artificial

<220>
<223>  membrane-vicinal region (MVR) of the extracellular
       membrane-proximal domain (EMPD)

<400>  14

Glu Leu Asp Val Cys Val Glu Glu Ala Glu Gly Glu Ala Pro
1               5                   10

<210>  15
<211>  19
<212>  PRT
<213>  Artificial

<220>
<223>  membrane-vicinal region (MVR) of the extracellular
       membrane-proximal domain (EMPD)

<400>  15

Glu Leu Asp Leu Gln Asp Leu Cys Ile Glu Glu Val Glu Gly Glu Glu
1               5                   10                  15

Leu Glu Glu

<210>  16
<211>  13
<212>  PRT
<213>  Artificial

<220>
<223>  membrane-vicinal region (MVR) of the extracellular
       membrane-proximal domain (EMPD)

<400>  16

Glu Gly Glu Val Ser Ala Asp Glu Glu Gly Phe Glu Asn
1               5                   10

<210>  17
<211>  27
<212>  PRT
<213>  Artificial

<220>
<223>  membrane-vicinal region (MVR) of the extracellular
       membrane-proximal domain (EMPD)

<400>  17

Tyr Leu Ala Met Thr Pro Leu Ile Pro Gln Ser Lys Asp Glu Asn Ser
1               5                   10                  15

Asp Asp Tyr Thr Thr Phe Asp Asp Val Gly Ser
            20                  25

<210>  18
<211>  18
<212>  PRT
<213>  Artificial

<220>
<223>  membrane-vicinal region (MVR) of the extracellular
       membrane-proximal domain (EMPD)

<400>  18

Glu Leu Gln Leu Glu Glu Ser Cys Ala Glu Ala Gln Asp Gly Glu Leu
1               5                   10                  15

Asp Gly

<210>  19
<211>  32
<212>  PRT
<213>  Artificial

<220>
<223>  membrane-vicinal region (MVR) of the extracellular
       membrane-proximal domain (EMPD)

<400>  19

Gly Ser Cys Ser Val Ala Asp Trp Gln Met Pro Pro Pro Tyr Val Val
1               5                   10                  15

Leu Asp Leu Pro Gln Glu Thr Leu Glu Glu Glu Thr Pro Gly Ala Asn
            20                  25                  30

<210>  20
<211>  26
<212>  PRT
<213>  Artificial

<220>
<223>  membrane-vicinal region (MVR) of the extracellular
       membrane-proximal domain (EMPD)

<400>  20

Asp Trp Gln Met Pro Pro Pro Tyr Val Val Leu Asp Leu Pro Gln Glu
1               5                   10                  15

Thr Leu Glu Glu Glu Thr Pro Gly Ala Asn
            20                  25

<210>  21
<211>  67
<212>  PRT
<213>  Artificial

<220>
<223>  membrane-vicinal region (MVR) of the extracellular
       membrane-proximal domain (EMPD)

<400>  21

Gly Leu Ala Gly Gly Ser Ala Gln Ser Gln Arg Ala Pro Asp Arg Val
1               5                   10                  15

Leu Cys His Ser Gly Gln Gln Gln Gly Leu Pro Arg Ala Ala Gly Gly
            20                  25                  30

Ser Val Pro His Pro Arg Cys His Cys Gly Ala Gly Arg Ala Asp Trp
        35                  40                  45

Pro Gly Pro Pro Glu Leu Asp Val Cys Val Glu Glu Ala Glu Gly Glu
    50                  55                  60

Ala Pro Trp
65


<210>  22
<211>  15
<212>  PRT
<213>  Artificial

<220>
<223>  membrane-vicinal region (MVR) of the extracellular
       membrane-proximal domain (EMPD)

<400>  22

Glu Leu Asp Val Cys Val Glu Glu Ala Glu Gly Glu Ala Pro Trp
1               5                   10                  15